# EUROPÄISCHE PATENTSCHRIFT

(11) **EP 0 929 294 B1**
(45) Veröffentlichungstag und Bekanntmachung des Hinweises auf die Patenterteilung: **25.06.2003**
(21) Anmeldenummer: 97921603.3
(22) Anmeldetag: 04.04.1997
(51) Int. Cl.: A61K 9/14, A61K 9/20

(54) **VERFAHREN ZUR HERSTELLUNG VON GERING DOSIERTEN FREIFLIESSENDEN UND/ODER DIREKTKOMPRIMIERBAREN PULVERSYSTEMEN**
PROCESS FOR PRODUCING SMALL DOSES OF FLUID AND/OR DIRECTLY COMPRESSIBLE POWDER SYSTEMS
PROCEDE DE PRODUCTION DE FAIBLES DOSES DE SYSTEMES PULVERULENTS FLUIDES ET/OU DIRECTEMENT COMPRESSIBLES

(30) Priorität: 04.04.1996 DE 19613545
(43) Veröffentlichungstag der Anmeldung: 21.07.1999
(73) Patentinhaber: Specht, Felix, 83533 Edling (DE)
(72) Erfinder: Specht, Felix, 83533 Edling (DE)
(74) Vertreter: Lang, Ulrich (DE)
(86) Internationale Anmeldenummer: DE9700691
(87) Internationale Veröffentlichungsnummer: WO97037638

(56) Entgegenhaltungen:
- EP-A- 0 277 741
- EP-A- 0 361 874
- WO-A-93/00991
- DE-A- 4 400 295
- US-A- 3 836 618

## Beschreibung

### Technisches Gebiet

Die Erfindung bezieht sich auf Verfahren zur Herstellung von gering dosierten freifließenden und/oder direktkomprimierbaren Pulversystemen.

Derartige Pulversysteme werden insbesondere zur Abfüllung von Pulverzubereitungen in Sachets oder zur Direkttablettierung verwendet, wobei abgeteilte Pulver oder Tabletten mit niedrigem Wirkstoffgehalt erhalten werden.

### Stand der Technik

Verfahren zur Herstellung von Zubereitungen mit niedrigem Wirkstoffgehalt, insbesondere zur Herstellung von Sachets oder Tabletten, sind seit langem bekannt.

Hierbei werden die zu verarbeitenden Wirkstoffe mit den Pulver- bzw. Tablettenfüllmitteln vermischt. Anschließend erfolgt die Zugabe von Fließregulierungsmitteln, sowie bei der Tablettenherstellung von Binde-, Spreng-, Trenn- und Schmiermitteln. Ebenso können noch andere Hilfsstoffe, wie beispielsweise Geschmackskorrigentien oder Farbstoffe zugesetzt werden.

Bei der direkten Verarbeitung dieser Mischungen zu abgeteilten Pulvern oder Tabletten erhält man aufgrund der während der Abfüllung oder der Verpressung zu Tabletten auftretenden Entmischungsprozesse Produkte mit unzureichender Dosierungsgenauigkeit. Diese Schwankungen des Wirkstoffgehalts wirken sich insbesondere bei Arzneiformen mit niedrig dosierten Wirkstoffen nachteilig aus, da sich bei den, bei diesen Arzneiformen auftretenden Differenzen zwischen tatsächlichem Wirkstoffgehalt und dem Sollwert größere prozentuale Abweichungen ergeben, als bei Arzneiformen mit höherem Wirkstoffgehalt. Darüber hinaus können bei niedrig zu dosierenden, stark wirksamen Stoffen oft nur sehr kleine Schwankungsbreiten in der Dosierung toleriert werden.

Zur Verringerung der Entmischung ist es üblich, die Mischungen von Wirk- und Füllstoffen vor der Weiterverarbeitung zu granulieren. Hierbei stehen grundsätzlich die bekannten Methoden der Feucht- und der Trokkengranulierung zur Verfügung:

Bei der Feuchtgranulierung, die beispielsweise in der BE-PS 773 064 beschrieben ist, wird die Wirk- und Füllstoffmischung erst mit einer Granulierflüssigkeit durchfeuchtet und dann granuliert. Das noch feuchte Granulat wird klassiert, anschließend getrocknet und dann nochmals klassiert.

Ebenso ist es möglich, den Wirkstoff in der Granulierflüssigkeit zu lösen oder zu dispergieren. Das Einbringen der Wirkstoffe erfolgt somit bei Zugabe der Granulierflüssigkeit zu den Füllstoffen und der Arbeitsschritt des Mischens von Wirk- und Füllstoffen entfällt bei diesem Verfahren.

Bei der Trockengranulierung werden die in einem vorangestellten Arbeitsschritt gemischten Bestandteile verpreßt, der Preßling wird in kleinere Teile gebrochen und anschließend werden die erhaltenen Granulatteile klassiert.

Die resultierenden Granulate haben bei einer nachfolgenden Abfüllung oder Verpressung zu Tabletten eine erheblich verminderte Entmischungsneigung.

Trotzdem weisen die bekannten Verfahren eine Reihe von Nachteilen auf:

Insbesondere erhält man bei dem obenbeschriebenen Verfahren der Trockengranulierung Produkte mit nicht ausreichender Homogenität des Wirkstoffgehalts der einzelnen Zubereitung.

Dies geht insbesondere auch aus EXAMPLE II der US 4 628 051 hervor, die die Tablettenherstellung aus β-Lactose und niedrig dosierten Steroiden beschreibt.

Ähnliche Verfahren werden in US 4 544 554, US 4 616 006 und US 4 530 839 beschrieben. Diese Verfahren, die ebenfalls zur Herstellung von Tabletten mit niedrigem Steroidgehalt verwendet werden, weisen jedoch auch ähnliche Dosierungsungenauigkeit auf.

Durch Feuchtgranulierung, die bei der Herstellung niedrig dosierter Arzneiformen beispielsweise in der US 3 939 264, US 3 969 502, US 4 425 339 und US 4 390 531 beschrieben ist, kann eine bessere Dosierungsgenauigkeit der mit dem erhaltenen Granulat hergestellten Arzneiformen erzielt werden. Ein Verfahren, das sich der Feuchtgranulierung als einem der Tablettierung vorangestellten Arbeitsschritt bedient, ist jedoch relativ zeitaufwendig, da viele einzelne Verfahrensschritte notwendig sind.

Ferner ist es bekannt, die Dosierungsgenauigkeit von niedrig dosierten Arzneiformen durch eine homogenere Verteilung des Wirkstoffs in dem Granulat, das zur Herstellung von abgeteilten Pulvern oder Tabletten verwendet wird, zu verbessern. Hierzu wird der niedrig zu dosierende Wirkstoff in einer Lösung auf ein Pulversystem aufgesprüht, das anschließend nach den bekannten Methoden granuliert wird. Auch dieses Verfahren zur Herstellung von niedrig dosierten Arzneiformen ist mit einem hohen Zeitaufwand verbunden, da kein Verfahrensschritt eingespart, sondern lediglich der Mischvorgang von Wirk- und Füllstoffen vor der Granulierung durch einen Sprühvorgang ersetzt wird.

Eine Verringerung der Verfahrensschritte und eine damit zu erreichende Zeitersparnis bei der Herstellung von niedrig dosierten Arzneiformen wermöglicht das in der EP 0 503 321 A1 beschriebene Verfahren auf. Hierbei werden zur Herstellung von gering dosierten freifließenden und/oder direktkomprimierbaren Pulversystemen Wirkstoffe in mikronisierter Form mit Hilfsstoffen gemischt. Nach diesem Mischvorgang werden weitere Hilfsstoffe, wie Fließ- und Schmiermittel zugegeben. Dann kann die Mischung entweder direkt verpreßt, in Kapseln oder Pulver abgeteilt oder zu einem Trockengranulat weiterverarbeitet werden.

Da bei diesem Verfahren die Hilfsstoffe in der Lage sein müssen, den Wirkstoff zu binden, um eine Entmischung von weniger als 10 % zu erreichen, ist das genannte Verfahren nur für Steroide in Kombination mit sprühgetrockneten Polyalkoholen, agglomerierter Lactose-Monohydrat, die auch unter dem Handelsnamen Tablettose bekannt ist, und Mischungen hieraus geeignet.

### Darstellung der Erfindung

Die Aufgabe der vorliegenden Erfindung ist es, ein Verfahren zur Herstellung von gering dosierten freifließenden und/oder direktkomprimierbaren Pulversystemen anzugeben, das zeitsparend durchzuführen ist und trotzdem eine Dosierungsgenauigkeit aufweist, die vergleichbar ist mit Arzneiformen, die durch Feuchtgranulierung oder durch das in der EP 0 503 521 A1 beschriebene Verfahren hergestellt werden. Insbesondere soll das erfindungsgemäße Verfahren in einem Direkttablettierungsprozeß einsetzbar sein.

Eine erfindungsgemäße Lösung dieser Aufgabe ist im Patentanspruch 1 angegeben. Weiterbildungen der Erfindung sind Gegenstand der Ansprüche 2 folgende.

Erfindungsgemäß wird ein Verfahren zur Herstellung von gering dosierten wirkstoffhaltigen Pulversystemen beschrieben, bei dem der Wirkstoff oder die Wirkstoffe aus einer Lösung und/oder Dispersion auf freifließende, und/oder direktkomprimierbare und insbesondere direkttablettierbare Pulversysteme versprüht wird.

Die Erfindung geht dabei von dem Grundgedanken aus, den oder die Wirkstoffe auf der Oberfläche von insbesondere direkt tablettierbaren Hilfsstoffen zu adsorbieren, d.h. im Kern befindet sich der Hilfsstoff und an der Oberfläche der Wirkstoff. Der Hilfsstoff stellt die Hauptkomponente der Darreichungsform dar, auf dessen Oberfläche der Wirkstoff sehr niedrig dosiert vorliegt. Damit eignet sich das erfindungsgemäße Verfahren insbesondere zur Herstellung von Arzneimitteln mit potenten Wirkstoffen, wie beispielsweise Hormonen.

Damit werden Wirkstoffe, die nicht homogen mit Hilfsstoffen mischbar sind, überhaupt erst direkt tablettierbar. Darüberhinaus wird der Wirkstoff auf der Oberfläche des Hilfsstoffs aufgrund des Sprühverfahrens homogen adorbiert.

Diese Vorgehensweise steht im Gegensatz zu dem aus der US-PS 3 907 983 bekannten Verfahren, bei dem die Wirkstoffe im Kern vorliegen und mit einem hydrophilen Polymer mittels eines Film-Coating-Systems überzogen und anschließend eventuell unter Beihilfe von Lactose direkt tablettiert werden.

Durch die erfindungsgemäße Vorgehensweise entfällt der Schritt der Feuchtgranulierung oder der Mischung von mikronisierten Wirkstoffen mit den Hilfsstoffen. Direkt anschließend an den Sprühvorgang kann das Pulversystem tablettiert oder zu Kapseln oder abgeteilten Pulvern verarbeitet werden.

Als freifließende und/oder direkttablettierbare Pulversysteme eignen sich für das erfindungsgemäße Verfahren Lactosen (wie z.B. agglomerierte, sprühgetrocknete oder kristalline Lactose), Cellulosen (wie z.B. mikrokristalline Cellulose, Cellulosepulver), Zucker (wie z.B.

Dextrose, Maltodextrin), Zuckeralkohole (wie z.B. Sorbit), Stärken (wie z.B. Maisstärke) und/oder Mischungen daraus, sogenannte Ein-Körper-Systeme aus zwei oder mehreren Hilfsstoffen oder Compounds (wie z.B. die Produkte mit den Handelsnamen Cellactose, Pharmatose DCL40, Microcelac 100, u.a.).

Der oder die Wirkstoffe können aus einer Lösung und/oder Dispersion auf diese Pulversysteme mit Hilfe verschiedener Sprühsysteme versprüht werden.

Als Lösungs- und/oder Dispersionsmittel sind beispielsweise Wasser oder organische Lösungsmittel oder Mischungen daraus geeignet.

Damit es zu einer verbesserten Haftung des Wirkstoffes oder der Wirkstoffe auf den Trägermaterialien kommt, kann die zu versprühende Zubereitung Bindemittel (wie z.B. Polyvinylpyrrolidon) enthalten.

Es können auf diese Weise Tabletten sowie Kapseln und abgeteilte Pulver, sogenannte Sachets, mit nahezu gleichen Wirkstoffmengen hergestellt werden.

Die Herstellung eignet sich für Tabletten und abgeteilte Arzneiformen mit einem Wirkstoffgehalt (bezogen auf die Masse der Gesamttablette) im allgemeinen von 0,0005% bis 5%, bevorzugt 0,005% bis 1 % und noch bevorzugter für Systeme mit 0,01 bis 0,5% Wirkstoffgehalt.

Es besteht die Möglichkeit des Versprühens einer großen Anzahl von Wirkstoffen auf die Pulversysteme. Im allgemeinen können folgende Wirkstoffgruppen auf die Trägermaterialien versprüht werden:
- hydroxylierte Kohlenwasserstoffe
- Carbonylverbindungen wie Ketone (z.B. Haloperidol), Monosaccharide, Disacharide und Aminozucker
- Carbonsäuren wie aliphatische Carbonsäuren, Ester aliphatischer und aromatischer Carbonsäuren (z.B. Atropin, Scopolamin), Lactone (z.B. Erythromycin), Amide und Imide aliphatischer Carbonsäuren, Aminosäuren, aliphatische Aminocarbonsäuren, Peptide, Polypeptide, β-Lactamderivate, Penicilline, Cephalosporine, aromatische Carbonsäuren (z.B. Acetylsalicylsäure), Amide aromatischer Carbonsäuren, vinyloge Carbonsäuren und Carbonsäureester
- Kohlensäureester wie Urethane und Thiourethane, Harnstoff und Harnstoffderivate, Guanidinderivate, Hydantoine, Barbitursäurederivate und Thiobarbitursäurederivate
- Nitroverbindungen wie aromatische Nitroverbindungen und heteroaromatische Nitroverbindungen
- Amine wie aliphatische Amine, Aminoglykoside, Phenylalkylamine, Ephedrinderivate, Hydroxyphenylethanolamine, Adrenalinderivate, Amfetaminderivate, aromatische Amine und quartäre Ammmoniumverbindungen
- schwefelhaltige Verbindungen wie Thiole und Disulfane, Sulfone, Sulfonsäureester und Sulfonsäureamide
- Polycarbocyclen wie Tetracycline, Steroide
- 0-haltige Heterocyclen wie Chromanderivate (z.B. Cromoglicinsäure)
- N-haltige Heterocyclen wie Pyrazolderivate (z.B. Propyphenazon, Phenylbutazon), Imidazolderivate (z.B. Histamin, Pilocarpin), Pyridinderivate (z.B. Pyridoxin, Nicotinsäure), Pyrimidinderivate (z.B. Trimetoprim), Indolderivate (z.B. Indometacin), Lysergsäurederivate (z.B. Ergotamin), Yohimbinderivate, Pyrroloindolderivate, Purinderivate (z.B. Allopurinol), Xanthinderivate, 8-Hydroxychinolinderivate, Amino-hydroxyalkylierte
   Chinoline, Aminochinoline, Isochinolinderivate (z.B. Morphin, Codein), Chinazolinderivate, Benzopyridazinderivate, Pteridinderivate (z.B. Methotrexat), 1,4-Benzodiazepinderivate, tricyclische N-haltige Heterocyclen, Acridinderivate (z.B. Ethacridin) und Dibenzazepinderivate (z.B. Trimipramin)
- S-haltige Heterocyclen wie Thioxanthenderivate (z.B. Chlorprothixen)
- N,O- und N,S-haltige Heterocyclen wie monocyclische N,O-haltige Heterocyclen, monocyclische N,S-haltige Heterocyclen, Thiadiazinderivate, bicyclische N,Shaltige Heterocyclen, Benzothiadiazinderivate, tricyclische N,S-haltige Heterocyclen und Phenothiazinderivate
- O,P,N-haltige Heterocyclen (z.B. Cyclophosphamid)
- pflanzliche Extrakte und/oder aus pflanzlichem und/oder tierischem Material hergestellte Wirkstoffe

Bevorzugte Wirkstoffe sind jedoch Steroide, insbesondere Ethinylestradiol, Gestoden, 3-Ketodesogestrel und Desogestrel, antibiotische, hormonell wirksame und/oder blutdrucksenkende Substanzen.

Besonders vorteilhaft ist auch die Verwendung des erfindungsgemäßen Verfahrens zur Besprühung von freifließenden und/oder direktkomprimierbaren Pulversystemen mit Süßstoffen und/oder Aromastoffen.

Hier sind als Pulversysteme beispielsweise Lactose, Maltodextrin, Stärke und/oder Polydextrosen oder Mischungen daraus besonders geeignet. Als Süßstoffe sind bevorzugt Aspartam, Saccharin bzw. seine Salze, Cyclamat bzw. seine Salze, Acesulfam, Stevioside, Glycyrrhizin, oder der unter der Handelsbezeichnung Talin erhältliche Süßstoff oder Mischungen daraus zu verwenden.

Bei der Herstellung von freifließenden und/oder direkttablettierbaren Pulversystemen mit gering dosierten Süßstoffen und/oder Aromastoffen ist die Abteilung in Sachets bevorzugt.

Das erfindungsgemäße Verfahren unterscheidet sich von den Verfahren, bei denen anschließend an das Versprühen der Wirkstoffzubereitung auf das Pulversystem eine Granulierung folgt durch den Wegfall dieses Granulierungsschrittes. Die Granulierprozesse beinhalten folgende Herstellungsschritte, die bei dem hier beanspruchten Verfahren, an das sich eine Direkttablettierung oder eine direkte Abfüllung des besprühten Pulversystems in Kapseln oder Sachets anschließt, nicht notwendig sind:
- Aggregieren bzw. Formen der gemischten Pulverpartikel durch Befeuchten (Es werden sog. Granulierflüssigkeiten, die Bindemittel enthalten zugegeben), unter Druck oder durch Erwärmen
- Trocknen der Feuchtgranulate
- Klassieren durch Sieben; die angestrebte Partikelgröße richtet sich nach den Anforderungen der Anwendung oder der Weiterverarbeitung.

Die Erfindung unterscheidet sich ebenfalls von Verfahren, bei denen der Wirkstoff oder die Wirkstoffe mit den direkttablettierbaren Pulversystemen gemischt werden, wie beispielsweise in der EP 0 503 521 A1 beschrieben.

Weiterhin unterscheidet sich die Erfindung von dem in der DE 35 05 424 A1 beschriebenen Verfahren, bei dem die Wirkstoffe nicht auf Trägermaterialien verklebt oder adsorbiert werden, sondern in deren Hohlräume eingebettet werden.

In jedem Falle erhält man durch das erfindungsgemäße Verfahren die Möglichkeit, niedrig dosierte Wirkstoffe, die nicht homogen mit den Hilfsstoffen mischbar sind, direkt zur oralen Applikation an Mensch oder Tier zu tablettieren, wobei die Konzentration der Wirkstoffe in jeder Tablette sehr konstant ist.

### Darstellung von Ausführungsbeispielen

Die Erfindung wird nachstehend ohne Beschränkung des allgemeinen Erfindungsgedankens anhand von Ausführungsbeispielen beschrieben:

### Beispiel 1:

| Bestandteile | Menge (mg pro Tablette) |
|---|---|
| Ethinylestradiol | 0,020 |
| Starch 1500 | 1,30 |
| Hochdisperses Siliciumdioxid | 0,80 |
| Magnesiumstearat | 0,40 |
| agglomerierte Lactose | ad 80,0 |

Ethinylestradiol wird in organischem Lösungsmittel gelöst und anschließend auf die Mischung von Stärke und Lactose versprüht. Nach dem Sprühvorgang werden dieser Zubereitung Fließregulierungsmittel und Schmiermittel zugesetzt, wonach die Tablettierung auf einer Tablettiermaschine erfolgen kann.

### Beispiel 2:

| Bestandteile | Menge (mg pro Tablette) |
|---|---|
| Desogestrol | 0,150 |
| Starch 1500 | 1,3 |
| Hochdisperses Siliciumdioxid | 0,80 |
| Magnesiumstearat | 0,40 |
| Fast Flow (sprühgetrocknete | |
| Lactose) | ad 80,0 |

Desogestrol wird in organischem Lösungsmittel gelöst und anschließend auf die Mischung von Stärke und Lactose versprüht. Nach dem Sprühvorgang werden dieser Zubereitung Fließregulierungsmittel und Schmiermittel zugesetzt, wonach die Tablettierung auf einer Tablettiermaschine erfolgen kann.

## Patentansprüche

1. Verfahren zur Herstellung von gering dosierten Tabletten oder Kapseln oder abgeteilten Pulvern, sogenannten Sachets,
**dadurch gekennzeichnet, daß**
wenigstens ein Wirkstoff aus einer Lösung oder Dispersion auf ein freifließendes und/oder direktkomprimierbares Pulversystem versprüht wird und das besprühte Pulversystem direkt zu den Tabletten oder zu den Kapseln oder zu den Sachets verarbeitet wird.

2. Verfahren nach Anspruch 1, **dadurch gekennzeichnet, daß** die Lösung oder Dispersion mit Hilfe verschiedener Sprühsysteme versprüht wird.

3. Verfahren nach einem der Ansprüche 1 bis 2, **dadurch gekennzeichnet, daß** als freifließende und/oder direkttablettierbare Pulversysteme Lactosen, Cellulosen, Zucker, Zuckeralkohole, Stärken und/oder Mischungen daraus verwendet werden.

4. Verfahren nach einem der Ansprüche 1 bis 3, **dadurch gekennzeichnet, daß** als Lösungs- oder Dispersionsmittel Wasser oder organische Lösungsmittel oder Mischungen daraus verwendet werden.

5. Verfahren nach einem der Ansprüche 1 bis 4, **dadurch gekennzeichnet, daß** die Wirkstofflösung oder -dispersion Bindemittel aufweist.

6. Verfahren nach einem der Ansprüche 1 bis 5, **dadurch gekennzeichnet, daß** der Wirkstoffgehalt der einzelnen Tabletten, Kapseln oder abgeteilten Pulver 0,0005 % bis 5 % beträgt.

7. Verfahren nach einem der Ansprüche 1 bis 5, **dadurch gekennzeichnet, daß** der Wirkstoffgehalt der einzelnen Tabletten, Kapseln oder abgeteilten Pulver 0,005 % bis 1 % beträgt.

8. Verfahren nach einem der Ansprüche 1 bis 5, **dadurch gekennzeichnet, daß** der Wirkstoffgehalt der einzelnen Tabletten, Kapseln oder abgeteilten Pulver 0,01 % bis 0,5 % beträgt.

9. Verfahren nach einem der Ansprüche 1 bis 8, **dadurch gekennzeichnet, daß** Steroide, antibiotische, hormonell wirksame und/oder blutdrucksenkende Wirkstoffe verwendet werden.

10. Verfahren nach einem der Ansprüche 1 bis 8, **dadurch gekennzeichnet, daß** als Wirkstoffe Süßstoffe und/oder Aromastoffe verwendet werden.

11. Verfahren nach Anspruch 10, **dadurch gekennzeichnet, daß** als Pulversystem Lactose, Maltodextrin, Stärke oder Polydextrosen oder Mischungen daraus verwendet werden.

12. Verfahren nach Anspruch 10 oder 11, **dadurch gekennzeichnet, daß** als Süßstoffe Aspartam, Acesulfam, Saccharin bzw. seine Salze, Cyclamat, Stevioside, Glycyrrhizin, oder der unter der Handelsbezeichnung Tallin erhältliche Süßstoff oder Mischungen daraus verwendet werden.

## Claims

1. Process for producing low-dose tablets or capsules or portioned powders, so-called sachets, **characterized in that** at least one active ingredient is sprayed from a solution or dispersion onto a free-flowing and/or directly compressible powder system, and the sprayed powder system is, without previously being dried, directly processed to the tablets, the capsules or the sachets.

2. Process according to Claim 1, **characterized in that** the solution or dispersion is sprayed with the aid of different spraying systems.

3. Process according to either of Claims 1 or 2, **characterized in that** lactoses, celluloses, sugars, sugar alcohols, starches and/or mixtures thereof are used as free-flowing and/or directly tabletable powder systems.

4. Process according to one or more of Claims 1 to 3, **characterized in that** water or organic solvents or mixtures thereof are used as solvents or dispersants.

5. Process according to one or more of Claims 1 to 4, **characterized in that** the active ingredient solution or dispersion comprises binders.

6. Process according to one or more of Claims 1 to 5, **characterized in that** the active ingredient content of the individual tablets, capsules or portioned powders is from 0.0005% to 5%.

7. Process according to one or more of Claims 1 to 5, **characterized in that** the active ingredient content of the individual tablets, capsules or portioned powders is from 0.005% to 1%.

8. Process according to one or more of Claims 1 to 5, **characterized in that** the active ingredient content of the individual tablets, capsules or portioned powders is from 0.01% to 0.5%.

9. Process according to one or more of Claims 1 to 8, **characterized in that** steroids, antibiotic, hormonally active and/or blood pressure-lowering active ingredients are used.

10. Process according to one or more of Claims 1 to 8, **characterized in that** sweeteners and/or flavourings are used as active ingredients.

11. Process according to Claim 10, **characterized in that** lactose, maltodextrin, starch or polydextroses or mixtures thereof are used as powder system.

12. Process according to either of Claims 10 or 11, **characterized in that** aspartame, acesulphame, saccharine or its salts, cyclamate, steviosides, glycyrrhizin or the sweetener talin, or mixtures thereof, are used as sweeteners.

## Revendications

1. Procédé pour la fabrication de comprimés, capsules ou poudres compartimentées, également appelées sachets, faiblement dosés, **caractérisé en ce qu'**au moins une substance active d'une solution ou dispersion est pulvérisée sur un système poudreux en écoulement libre et/ou directement compressible et **en ce que** le système poudreux pulvérisé est directement transformé, sans séchage préalable, en comprimés, capsules ou sachets.

2. Procédé selon la revendication 1, **caractérisé en ce que** la solution ou dispersion est pulvérisée à l'aide de différents systèmes de pulvérisation.

3. Procédé selon l'une des revendications 1 ou 2, **caractérisé en ce que** les systèmes poudreux en écoulement libre et/ou directement compressibles utilisés sont des lactoses, celluloses, sucres, alcools de sucre, amidons et/ou des mélanges de ces derniers.

4. Procédé selon l'une ou plusieurs des revendications 1 à 3, **caractérisé en ce que** les solvants ou dispersants utilisés sont de l'eau ou des solvants organiques ou des mélanges de ceux-ci.

5. Procédé selon l'une ou plusieurs des revendications 1 à 4, **caractérisé en ce que** la solution ou dispersion de substances actives contient des liants.

6. Procédé selon l'une ou plusieurs des revendications 1 à 5, **caractérisé en ce que** la teneur de substances actives des différents comprimés, capsules ou poudres compartimentées est de 0,0005 % à 5 %.

7. Procédé selon l'une ou plusieurs des revendications 1 à 5, **caractérisé en ce que** la teneur de substances actives des différents comprimés, capsules ou poudres compartimentées est de 0,005% à 1%.

8. Procédé selon l'une ou plusieurs des revendications 1 à 5, **caractérisé en ce que** la teneur de substances actives des différents comprimés, capsules ou poudres compartimentées est de 0,01 % à 0,5 %.

9. Procédé selon l'une ou plusieurs des revendications 1 à 8, **caractérisé en ce que** des stéroïdes, des substances actives antibiotiques, à effet hormonal et/ou régulateur de la pression artérielle sont utilisés.

10. Procédé selon l'une ou plusieurs des revendications 1 à 8, **caractérisé en ce que** les substances actives utilisées sont des édulcorants et/ou des substances aromatiques.

11. Procédé selon la revendication 10, **caractérisé en ce que** le système poudreux utilisé est du lactose, malto-dextrine, amidon ou polydextroses ou des mélanges de ceux-ci.

12. Procédé selon l'une des revendications 10 ou 11, **caractérisé en ce que** les édulcorants utilisés sont aspartame, acésuffame, saccharine ou ses sels, cyclamate, stévioside, glycyrrhizine ou un édulcorant commercialisé sous le nom de Tal in ou des mélanges de ceux-ci.
